Europäisches Patentamt

European Patent Office

Office européen des brevets

(19)

(11) Veröffentlichungsnummer: **0 254 956**

**A2**

(12)  **EUROPÄISCHE PATENTANMELDUNG**

(21) Anmeldenummer: **87110203.4**

(22) Anmeldetag: **15.07.87**

(51) Int. Cl.⁴: **C07C 2/20** , B01J 27/06

(30) Priorität: **29.07.86 DE 3625572**
    **29.07.86 DE 3625571**

(43) Veröffentlichungstag der Anmeldung:
    **03.02.88 Patentblatt  88/05**

(84) Benannte Vertragsstaaten:
    **DE FR GB**

(71) Anmelder: **Linde Aktiengesellschaft**
    **Abraham-Lincoln-Strasse 21**
    **D-6200 Wiesbaden(DE)**

(72) Erfinder: **Zimmermann, Heinz, Dr.-Ing.**
    **Herterichstrasse 83**
    **D-8000 München 71(DE)**

(74) Vertreter: **Schaefer, Gerhard, Dr.**
    **Linde Aktiengesellschaft Zentrale**
    **Patentabteilung**
    **D-8023 Höllriegelskreuth(DE)**

(54)  **Verfahren zur Oligomerisierung von Olefinen.**

(57) Es wird ein Verfahren zur Oligomerisierung von
Olefinen beschrieben. Um Umweltverschmutzungs-
und Korrosionsprobleme zu vermeiden, wird vorgeschlagen, daß die Oligomerisierung in der Gasphase
an einem festen Katalysator, der supersaure Fluorverbindungen der Elemente der III., IV. und/oder V.
Hauptgruppe des Periodensystems enthält, durchgeführt wird. Das Verfahren kann beispielsweise zur
Erhöhung der Olefinausbeute in Steamcrackern oder
zur Gewinnung von Kraftstoffkomponenten aus FCC-
Abgasen eingesetzt werden.

EP 0 254 956 A2

## Verfahren zur Oligomerisierung von Olefinen

Die Erfindung betrifft ein Verfahren zur Oligomerisierung von Olefinen.

Auf vielen Gebieten der Polymerisationstechnik ist es zur Erreichung bestimmter Produktqualitäten wünschenswert, die Polymerisation auf einer niedrigen Polymerisationsstufe anzuhalten und eine Durchpolymerisierung zu vermeiden. Derartige Oligomerisierungen stellen daher zur Gewinnung bestimmter Produkte oft wichtige Reaktionen dar, deren gezielte Steuerung es verfahrenstechnisch zu beherrschen gilt.

Bei der Olefingewinnung in Steamcrackern sind die Ausbeuten der einzelnen Olefine aufgrund der Thermodynamik bzw. der Reaktionskinetik vorgegeben. Eine wesentliche Erhöhung der Ausbeute eines bestimmten Olefins bei konstanter Spaltschärfe ist bekanntlich nur durch Rückführung von Produkten erreichbar. Bei der thermischen Spaltung von kohlenwasserstoffhaltigen Einsätzen entstehen jedoch neben Ethylen und Propylen auch Olefine, wie Butene, die nicht zurückgeführt werden können, da sie zu einer raschen Verkokung der Spaltschlangen im Streamcracker führen würden. Dies bedeutet einen Verlust an Kohlenwasserstoffen.

Ein weiterer Anwendungsfall für Reaktionen der eingangs geschilderten Art findet sich bei den Abgasen von Verfahren des "fluid catalytic cracking" (im folgenden kurz "FCC" genannt). FCC-Abgase werden in aufwendigen Verfahren durch Alkylierung bzw. Polymerisierung in der Flüssigphase zu Kraftstoffkomponenten umgesetzt. Diese Komponenten erlangen im Zuge der Einführung bleifreien Benzins wachsende Bedeutung als Blendkomponenten. Es ist bekannt, diese Polymerisierungen als Oligomerisierungen durchzuführen. Dazu werden die verflüssigten FCC-Abgase in einem Fraktionierturm unter Druck von den leichten Anteilen befreit und in einer Reaktionskammer an einem Katalysator, vor allem Schwefelsäure, Phosphorsäure oder Fluorwasserstoff, umgesetzt. Die Reaktionsprodukte werden in einem weiteren Fraktionierturm in Polymerisat bzw. Alkylat als Bodenprodukt und leichte Anteile als Kopfprodukt zerlegt. Das Sumpfprodukt wird auf Siedegrenzen fraktioniert, die Abgase kehren in den Prozeß zurück.

Nachteile der bisher bekannten Verfahren sind, daß die verwendeten Säuren wieder mit aufwendigen Verfahren entfernt werden müssen und zum Teil erhebliche Umwelt-und Korrosionsprobleme verursachen. Bei Einsatz von gelöten Katalysatoren (Homogen-Katalyse) müssen die gelösten Katalysatoren aus dem Produkt wieder abgetrennt werden.

Der Erfindung liegt daher die Aufgabe zugrunde, ein Verfahren zu schaffen, das, unabhängig von der Art der Begleitkomponenten, eine gezielte Oligomerisierung von Olefinen ermöglicht.

Diese Aufgabe wird erfindungsgemäß dadurch gelöst, daß die Oligomerisierung in der Gasphase an einem festen Katalysator, der supersaure Fluorverbindungen der Elemente der III., IV. und/oder V. Hauptgruppe des Periodensystems enthält, durchgeführt wird.

In vorteilhafter Weise wird ein Katalysator eingesetzt, der aus einem $Al_2O_3$-Träger mit 0,1 bis 30 Gew.%, vorzugsweise 5 bis 15 Gew.%, supersauren Fluorverbindungen besteht.

Als supersaure Fluorverbindungen kommen alle Säuren in Frage, die Elemente der III., IV. und/oder V. Hauptgruppe des Periodensystems der Elemente enthalten. Als Vertreter derartiger Supersäuren seien $HBF_4$, $H_2SiF_6$ und $HPF_6$ genannt. Mit gleichermaßen guten Ergebnissen können auch Salze dieser Supersäuren verwendet werden, wie beispielsweis $NH_4BF_4$ oder $(NH_4)_2SiF_6$. Es liegt im Rahmen der Erfindung, daß auch andere als die speziell genannten Säuren bzw. Salze zur Anwendung gelangen können.

Der erfindungsgemäß für die Oligomerisierung eingesetzte Katalysator wird in an sich bekannter Weise durch Tränken des Trägers mit der Supersäure oder mit wäßrigen Lösungen von deren Salzen und nachfolgende Calcinierung hergestellt, wobei die supersaure Komponente chemisch an den Träger gebunden wird.

Aufgrund der Trägerfixierung der Supersäure sind die Reaktionsmedien nicht korrosiv, was zusammen mit milden Reaktionsbedingungen zu günstigen Investitionskosten führt.

Unter Supersäuren werden dabei solche Säuren verstanden, die stärker sauer sind als eine 100%ige Schwefelsäure. Supersäuren besitzen in der Regel einen pH-Wert von etwa -0,5.

Die Auswahl von supersauren Fluorverbindungen der Elemente der III., IV. und/oder V. Hauptgruppe des Periodensystems hat sich als besonders günstig erwiesen, da mit einem derartig supersauren Katalysator bei der Oligomerisierung von Olefinen hervorragende Ausbeuten bei hoher Selektivität und milden Reaktionsbedingen erzielt werden.

In Weiterbildung des Erfindungsgedankens wird die Oligomeriserung bei einem Druck zwischen 1 und 80 bar, vorzugsweise 5 und 50 bar, bei einer Temperatur zwischen 100 und 400°C, vorzugsweise 100 und 300°C, und einer Raumgeschwindigkeit ("weight hourly space velocity", im folgenden kurz "WHSV" genannt) zwischen 0,1 und 5 g / g.h durchgeführt.

Das erfindungsgemäße Verfahren ermöglicht eine gezielte Oligomerisierung von Olefinen, insbesondere Butenen, und kann somit außerordentlich vorteilhaft beispielsweise zur Gewinnung von Olefinen durch thermische Spaltung von kohlenwasserstoffhaltigen Einsätzen, wobei zur Erhöhung der Olefinausbeute Produkte zum Teil zur Spaltung zurückgeführt werden, und zur Gewinnung von Kraftstoffkomponenten durch Oligomerisierung von FCC-Abgasen eingesetzt werden.

Untersuchungen an Steamcrackern zur Herstellung von Ethylen und Propylen haben ergeben, daß durch Oligomerisierung bzw. Polymerisierung, insbesondere Di-, Tri-und Tetramerisierung, bei der Spaltung entstehende Butene in rückführbare Produkte umgewandelt werden können, die nicht zu Verlegungen des Steamcrackers führen. Insgesamt erhöht sich durch Oligomerisierung und Rückführung der Butene die Olefinausbeute, d.h. die Ausbeute an Ethylen und Propylen, um ca. 2 Gew.%.

Bei der Spaltung von Naphtha bzw. Gasöl entstehen bei üblichen Spaltbedingungen ca. 5 bis 6 Gew.% Butene. Diese können nach der Erfindung durch supersaure Katalysatoren mit ca. 90% Ausbeute dimerisiert werden. Bei einer durchschnittlichen Ethylenausbeute von 25% aus dem so erzeugten Dimer/Trimerbenzin bedeutet dies eine Erhöhung der Gesamtethylenausbeute um 5x0,9x0,25 = 1,125 Gew.% bis 6x0,9x0,25 = 1,35 Gew.% und für Propylen bei einem Verhältnis Propylen/Ethylen von 0,6 um 0,8 Gew.%.

Neben der Oligomerisierung von Butenen bewirkt der erfindungsgemäße Katalysator auch die Isomerisierung von n-Butenen zu i-Buten. Diese Isomerisierung führt zu stark verzweigten Produkten hoher Klopffestigkeit.

Nach einer zweckmäßigen Ausführungsform wird Butadien vor der Oligomerisierung aus dem butenhaltigen Strom in an sich bekannter Weise extrahiert.

Mit dem erfindungsgemäßen Verfahren ist eine Erhöhung der Ausbeute an Ethylen und Propylen bei Steamcrackern ohne Änderungen bzw. Umbauten an den Spaltöfen erreichbar.

Überdies können die C4-Buten-Dimeren bzw. -Trimeren alternativ als hochoktanige Kraftstoffkomponenten eingesetzt werden.

Wie bereits vorstehend erwähnt, können auch in FCC-Abgasen enthaltene Olefine mit Hilfe des erfindungsgemäßen Verfahrens oligomerisiert werden. Auf diese Weise können FCC-Abgase zu hochoktanigen Blendkomponenten verarbeitet werden.

C4-FCC-Abgase bestehen zum überwiegenden Teil, etwa 70%, aus Olefinen (10 bis 15 Gew.% aus i-Buten und der Rest aus Buten -1, cis-und trans-Buten-2) sowie n-und i-Butan. Oligomerisiert werden mit dem erfindungsgemäßen Verfahren die Butene, wobei eine gleichzeitige Isomerisierung der n-Butene zu i-Buten Produkte mit höheren Oktanzahlen ergeben.

Im Prüfmotor ergeben sich eine MOZ (Motor Oktanzahl) bis zu 85 und eine ROZ (Research Oktanzahl) bis zu 100. Die Oligomerbenzine zeigen bei der Mischung mit bleifreiem Normalbenzin Blendoktanzahlen bis 89 MOZ und 108 ROZ. Die Erfindung sei nunmehr anhand zweier Zahlenbeispiele noch näher erläutert.

Beispiel 1:

Über 150 ml eines supersauren Katalysators, der zum Beispiel aus einem A1203-Träger mit 10 Gew.% HBF4 besteht, wird ein butadienfreier C4-Schnitt aus einem Steamcracker (Raffinat 1) bei 300°C und 5 bar mit einer Raumgeschwindigkeit WHSV von 0,66 g/g.h geleitet. Über dem Katalysator findet bei den gegebenen milden Reaktionsbedingungen die Oligomerisierung der Butene statt. Das Produkt wird in einer Kühlfalle kondensiert und analysiert.

Aus der Analyse des Kondensates und des Kondensatorabgases errechnet sich ein Umsatz von 90,5%, bezogen auf Butene. Im einzelnen besteht das Oligomerisierungsprodukt zu 71,0 Gew.% aus Dimeren und zu 28,5 Gew.% aus Trimeren. Überdies sind noch Spuren von Tetrameren feststellbar.

Das Produkt kann zur thermischen Spaltung zurückgeführt werden, wodurch sich die Ausbeute an Ethylen und Propylen erhöht.

Beispiel 2:

Ein technischer FCC-C4-Schnitt (16 Gew.% Buten-1, 18 Gew.% i-Buten, 33 Gew.% cis/trans-Buten-2, 33 Gew.% i/n-Buten) werden bei 10 bar, 250°C und einer WHSV von 0,66 g/g.h umgesetzt. Die Produkte werden entspannt und in einem Abscheider in Gas und Flüssigprodukt getrennt. Aus der Analyse und den Mengenströmen von Gas-und Flüssigphase errechnet sich ein Umsatz von 64 Gew.%, bezogen auf die im Einsatz enthaltenen Olefine.

Die Produktfraktion 90 - 200°C (Dimere und Trimere) zeigt im Prüfmotor eine Blendoktanzahl auf bleifreies Normalbenzin von 104 ROZ und 86,6 MOZ.

**Ansprüche**

1. Verfahren zur Oligomerisierung von Olefinen, dadurch gekennzeichnet, daß die Oligomerisierung in der Gasphase an einem festen Katalysator, der supersaure Fluorverbindungen der Elemente der III., IV. und/oder V. Hauptgruppe des Periodensystems enthält, durchgeführt wird.

2. Verfahren nach Anspruch 1, dadurch gekennzeichnet, daß ein Katalysator, bestehend aus einem A1203-Träger mit 0,1 bis 30 Gew.%, vorzugsweise 5 bis 15 Gew.%, supersauren Fluorverbindungen verwendet wird.

3. Verfahren nach Anspruch 1 oder 2, dadurch gekennzeichnet, daß als supersaure Fluorverbindungen $HBF_4$, $NH_4BF_4$, $H_2SiF_6$, $(NH_4)_2SiF_6$ oder $HPF_6$ verwendet werden.

4. Verfahren nach einem oder mehreren der Ansprüche 1 bis 3, dadurch gekennzeichnet, daß die Oligomerisierung bei einem Druck zwischen 1 und 80 bar, vorzugsweise 5 und 50 bar, einer Temperatur zwischen 100 und 400°C, vorzugsweise 100 bis 300°C, und einer Raumgeschwindigkeit (WHSV) zwischen 0,1 bis 5 g/g.h durchgeführt wird.

5. Verfahren nach einem oder mehreren der Ansprüche 1 bis 4 zur Gewinnung von Olefinen durch thermische Spaltung von kohlenwasserstoffhaltigen Einsätzen, bei dem zur Erhöhung der Olefinausbeute Produkte zum Teil zur Spaltung zurückgeführt werden, dadurch gekennzeichnet, daß bei der Spaltung entstehende Butene in der Gasphase an dem die supersauren Fluorverbindungen enthaltenden Katalysator oligomerisiert und zur Spaltung zurückgeführt werden.

6. Verfahren nach Anspruch 5, dadurch gekennzeichnet, daß vor der Oligomerisierung Butadien extrahiert wird.

7. Verfahren nach einem oder mehreren der Ansprüche 1 bis 4 zur Gewinnung von Kraftstoffkomponenten durch Oligomerisierung von FCC-Abgasen, dadurch gekennzeichnet, daß die Oligomerisierung in der Gasphase an dem die supersauren Fluorverbindungen enthaltenden Katalysator durchgeführt wird.